# EUROPÄISCHE PATENTSCHRIFT

(11) **EP 4 051 194 B1**
(45) Veröffentlichungstag und Bekanntmachung des Hinweises auf die Patenterteilung: **16.08.2023**
(21) Anmeldenummer: 20799680.2
(22) Anmeldetag: 28.10.2020
(51) Int. Cl.: A61F 9/007

(54) **OPHTHALMOCHIRURGISCHE STEUERUNGSMODULVORRICHTUNG**
OPHTHALMO-SURGICAL CONTROL MODULE ASSEMBLY
ENSEMBLE MODULE DE COMMANDE DE CHIRURGIE OPHTHALMIQUE

(30) Priorität: 29.10.2019 DE 102019216670
(43) Veröffentlichungstag der Anmeldung: 07.09.2022
(73) Patentinhaber: Carl Zeiss Meditec AG, 07745 Jena (DE)
(72) Erfinder: KÜBLER, Christoph, 73447 Oberkochen (DE); KOHLHAMMER, Susanne, 89134 Blaustein (DE); NEUMAIER, Markus, 73432 Aalen (DE)
(74) Vertreter: Carl Zeiss AG - Patentabteilung
(86) Internationale Anmeldenummer: PCT/EP2020/080249
(87) Internationale Veröffentlichungsnummer: WO 2021/083939

(56) Entgegenhaltungen:
- EP-A2- 1 765 190
- WO-A2-2005/092022
- US-A1- 2005 209 561

## Beschreibung

Die Erfindung betrifft eine ophthalmochirurgische Steuerungsmodulvorrichtung.

Zur Behandlung einer Augenlinsentrübung, welcher in der Medizin als grauer Star bezeichnet wird, gibt es mehrere chirurgische Techniken. Die am weitesten verbreitete Technik ist die Phakoemulsifikation, bei der eine dünne Hohlnadel in die Augenlinse eingeführt und zu Ultraschallschwingungen angeregt wird. Die vibrierende Hohlnadel emulsifiziert in ihrer nächsten Umgebung die Linse derart, dass die entstehenden Linsenpartikel durch eine Leitung mittels einer Pumpe abgesaugt werden können. Dabei wird ein Spülfluid (Irrigationsfluid) zugeführt, wobei das Absaugen der Linsenpartikel und des Fluides durch eine Aspirationsfluidleitung erfolgt. Ist die Linse vollständig emulsifiziert und entfernt worden, kann in den leeren Kapselsack eine neue künstliche Linse eingesetzt werden, sodass ein derart behandelter Patient wieder ein gutes Sehvermögen erreichen kann.

Bei der Zerkleinerung der Augenlinse durch eine mit Ultraschall schwingende Hohlnadel ist es unvermeidbar, dass während der Operation ein relativ großes Linsenpartikel so vor der Spitze der Hohlnadel zur Anlage kommt, dass diese Nadelspitze bzw. ihre Absaugöffnung verstopft wird. Dieser Zustand wird als Okklusion bezeichnet. In einem solchen Fall baut eine Pumpe in der Aspirationsfluidleitung einen mehrfach stärkeren Saugdruck im Vergleich zu einem okklusionsfreien Betrieb auf. Zusätzlich kann ein starker Energieeintrag für die Bewegung der Hohlnadel erfolgen, sodass das die Hohlnadel verstopfende Linsenpartikel zertrümmert wird. Alternativ kann auch eine Umkehrung der Laufrichtung der Pumpe das Linsenpartikel von der Nadelspitze wieder entfernen, sodass wieder ein übliches Absaugen des Fluides und der kleinen Linsenpartikel erfolgen kann. In einem solchen Moment wird eine Okklusion somit aufgebrochen, wobei der zuvor anliegende hohe Unterdruck sehr schnell abnimmt. Der dadurch entstehende Sog kann dazu führen, dass nicht nur kleine Linsenpartikel und Fluid zur Aspirationsleitung gezogen werden, sondern auch ein Teil des Kapselsackes mit der Hohlnadel in Kontakt kommt. Wenn der Kapselsack durchstochen wird, führt dies zu erheblichen Komplikationen für den Patienten, welche unbedingt vermieden werden müssen.

Zudem kann bei dem Sog eine große Fluidmenge aus der Vorderkammer des Auges abgesaugt werden, sodass die Gefahr besteht, dass das Auge kollabiert. Auch dies kann zu erheblichen Komplikationen für den Patienten führen, welche unbedingt vermieden werden müssen.

Der Beginn einer Okklusion oder das Aufbrechen einer Okklusion kann durch Erfassen von Messsignalen in einem ophthalmochirurgischen System erkannt werden. Zum Beispiel kann ein Druck oder ein Volumenstrom in einer Irrigationsfluidleitung oder einer Aspirationsfluidleitung erfasst werden. Besonders geschickt ist es, wenn jeweils die Ableitung eines Druckverlaufes oder Volumenstromes über die Zeit vorgenommen wird, da somit schnell eine Änderung des Signalverlaufes erkannt werden kann. Ein Signalverlauf für Druck und Volumenstrom weist jedoch stets ein Rauschen auf, sodass eine Ableitung eines solchen Signals zu hohen Ausschlägen führen kann. Damit ist die Wahrscheinlichkeit relativ hoch, dass eine angeblich signifikante Druckänderung oder Volumenstromänderung falsch gedeutet wird. Eine auf einer solchen Basis ermittelte Zustandsbeschreibung eines ophthalmochirurgischen Systems kann zu hektischen Schaltvorgängen und damit zu Komplikationen während einer Phakoemulsifikation führen. Dokument EP1765190 stellt den Stand der Technik dar und offenbart ein chirurgisches System, zum Detektieren des Beginns einer Okklusion oder des Aufbrechens einer Okklusion durch Erfassen von Messsignalen.

Es ist eine Aufgabe der Erfindung, eine ophthalmochirurgische Steuerungsmodulvorrichtung zu schaffen, mit welcher die Wahrscheinlichkeit für eine medizinische Komplikation oder eine Verletzungsgefahr während einer Phakoemulsifikation verringert werden kann. Es ist eine weitere Aufgabe der Erfindung, ein ophthalmochirurgisches System mit einer solchen Steuerungsmodulvorrichtung zu schaffen.

Die Aufgabe wird für die ophthalmochirurgische Steuerungsvorrichtung durch den Gegenstand des unabhängigen Patentanspruchs 1 gelöst. Vorteilhafte Weiterbildungen der Erfindung sind Gegenstand der Unteransprüche. Die Aufgabe wird für das ophthalmochirurgische System durch den Gegenstand des nebengeordneten Patentanspruchs gelöst.

Gemäß der Erfindung weist die ophthalmochirurgische Steuerungsmodulvorrichtung, auf:
- eine erste Faltungsfunktionsvorrichtung, welche eingerichtet ist, an ihrem Eingang ein an einer Irrigationsfluidleitung erfasstes erstes Messsignal von einer ersten Messvorrichtung zu empfangen, und an ihrem Ausgang einen mittels einer ersten Faltungsfunktion gefalteten Betrag einer zeitlichen Ableitung des ersten Messsignals auszugeben, wobei die erste Faltungsfunktion eine erste Zeitdauer besitzt,
- eine erste Schwellwertvorrichtung, mit welcher sich ein erster Schwellwert für die zeitliche Ableitung des ersten Messsignals einrichten lässt,
- eine erste Vergleichsvorrichtung, welche eingerichtet ist, den von der ersten Faltungsfunktionsvorrichtung ausgegebenen Betrag der zeitlichen Ableitung des ersten Messsignals zu empfangen und den ersten Schwellwert zu empfangen und nach Ablauf eines vorbestimmten ersten Zeitpunktes den ausgegebenen Betrag der zeitlichen Ableitung des ersten Messsignals und den ersten Schwellwert miteinander zu vergleichen und ein erstes Vergleichsergebnis auszugeben,
- eine zweite Faltungsfunktionsvorrichtung, welche eingerichtet ist, an ihrem Eingang das an der Irrigationsfluidleitung erfasste erste Messsignal zu empfangen, und an ihrem Ausgang einen mittels einer zweiten Faltungsfunktion gefalteten Betrag einer zeitlichen Ableitung des ersten Messsignals auszugeben, wobei die zweite Faltungsfunktion eine zweite Zeitdauer besitzt, welche kürzer als die erste Zeitdauer ist,
- eine zweite Schwellwertvorrichtung, mit welcher sich ein zweiter Schwellwert für die zeitliche Ableitung des ersten Messsignals einrichten lässt,
- eine zweite Vergleichsvorrichtung, welche eingerichtet ist, den von der zweiten Faltungsfunktionsvorrichtung ausgegebenen Betrag der zeitlichen Ableitung des ersten Messsignals zu empfangen und den zweiten Schwellwert zu empfangen und nach Ablauf eines vorbestimmten zweiten Zeitpunktes den ausgegebenen Betrag der zeitlichen Ableitung des ersten Messsignals und den zweiten Schwellwert miteinander zu vergleichen und ein zweites Vergleichsergebnis auszugeben,
- eine erste Auswertevorrichtung, welche eingerichtet ist, das erste Vergleichsergebnis und das zweite Vergleichsergebnis zu empfangen und auszuwerten und ein erstes Auswertesignal auszugeben,
- eine Ansteuerungsvorrichtung, welche eingerichtet ist, das erste Auswertesignal zu empfangen und in Abhängigkeit von dem ersten Auswertesignal ein Steuersignal zur Steuerung eines Parameters einer ophthalmochirurgischen Einrichtung bereitzustellen.

Eine Faltungsfunktionsvorrichtung ist eine Vorrichtung, welche eingerichtet ist, mittels einer Faltungsfunktion eine Faltung von Daten vorzunehmen. Eine Faltung oder Konvolution ist ein mathematischer Operator, der aus zwei Funktionen eine dritte Funktion ("gefaltete Funktion") erzeugt. Die Steuerungsmodulvorrichtung ist somit geeignet, ein eine erste Funktion bildendes Messsignal mit einer Faltungsfunktion einer Faltungsfunktionsvorrichtung zu falten. Die erste Faltungsfunktion besitzt eine erste Zeitdauer. Dies bedeutet, dass Messwerte des Messsignals, welche während einer ersten Zeitdauer zur Verfügung stehen, mit der Faltungsfunktion gefaltet werden. Dies können zum Beispiel die letzten 100 Messwerte des Messsignals sein, die vor der Faltung zur Verfügung standen. Die zweite Faltungsfunktion besitzt eine zweite Zeitdauer, welche kürzer als die erste Zeitdauer ist. Dies bedeutet, dass Messwerte des Messsignals, welche während der zweiten Zeitdauer zur Verfügung stehen, mit der zweiten Faltungsfunktion gefaltet werden. Da die zweite Zeitdauer kürzer als die erste Zeitdauer ist, werden von der zweiten Faltungsfunktion weniger Messwerte des Messsignals berücksichtigt und gefaltet. Dies können zum Beispiel die letzten 10 Messwerte des Messsignals sein, die vor der Faltung zur Verfügung standen.

Gemäß der Erfindung bewirkt die erste Faltungsfunktion und auch die zweite Faltungsfunktion, dass am Ausgang der ersten und zweiten Faltungsfunktionsvorrichtung jeweils ein Betrag einer zeitlichen Ableitung des ersten Messsignals ausgegeben wird. Wenn ein Messsignal eine Änderung in seinem Verlauf erfährt, kann dies mittels einer Ableitung des Signalverlaufes schnell und eindeutig erkannt werden. Indem einer erste Faltungsfunktionsvorrichtung und eine zweite Faltungsfunktionsvorrichtung mit jeweiligen Faltungsfunktionen unterschiedlicher Zeitdauer zum Einsatz kommen, ist es möglich, unter Berücksichtigung eines jeweiligen Schwellwertes und Vergleich der gefalteten Signale mit den jeweiligen Schwellwerten zu erkennen, ob das Messsignal ein Störsignal oder Rauschsignal aufweist und daher auch keine Okklusion oder kein Aufbrechen einer Okklusion vorliegt. Genauso ist es möglich, das Fehlen eines solchen Störsignals oder Rauschsignals zu erkennen, sodass eine schnelle und insbesondere zuverlässige Detektion einer Okklusion oder das Aufbrechen einer Okklusion möglich ist. In Abhängigkeit von dem ersten Auswertesignal kann dann die Ansteuerungsvorrichtung ein Steuersignal zur Steuerung eines Parameters der ophthalmochirurgischen Einrichtung bereitstellen. Ein solcher Parameter kann ein von einer Fluidpumpe erzeugter Druck in einer Irrigationsfluidleitung oder Aspirationsfluidleitung sein, oder ein von einer Fluidpumpe bewirkter Volumenstrom in einer Irrigationsfluidleitung oder Aspirationsfluidleitung, oder eine geänderte Energiezufuhr zu Piezokeramiken eines chirurgischen Handstückes. Auf diese Weise kann die Wahrscheinlichkeit für eine medizinische Komplikation oder eine Verletzungsgefahr während einer Phakoemulsifikation verringert werden.

Gemäß einer Weiterbildung der Erfindung sind die erste Faltungsfunktion und die zweite Faltungsfunktion jeweils punktsymmetrisch zu ihrem jeweiligen Koordinatenursprung. Dies ist vorteilhaft, da es somit nach der Faltung zu keiner Verschiebung des Absolutbetrages des ausgegebenen Wertes oder Bevorzugung einzelner Messwerte des Messsignals kommt. Ferner ist eine Punktsymmetrie vorteilhaft für die Genauigkeit der ermittelten Ableitung des ersten Messsignals.

Bevorzugt ist der Betrag des zweiten Schwellwertes verschieden von dem Betrag des ersten Schwellwertes. Besonders bevorzugt ist der Betrag des zweiten Schwellwertes höher als der Betrag des ersten Schwellwertes. Der zweite Schwellwert wird mit einem Betrag der zweiten Faltungsfunktionsvorrichtung verarbeitet, welche eine Faltungsfunktion mit einer zweiten Zeitdauer aufweist, welche kürzer als die erste Zeitdauer ist. Wenn der zweite Schwellwert höher als der erste Schwellwert ist, muss somit der von der zweiten Faltungsfunktionsvorrichtung ausgegebene Betrag einen höheren Betrag aufweisen als der von der ersten Faltungsfunktionsvorrichtung ausgegebene Wert, um einen Schnittpunkt mit dem zweiten Schwellwert zu erreichen. Da die zweite Zeitdauer kürzer als die erste Zeitdauer ist, werden weniger Messwerte als bei der ersten Faltungsfunktion berücksichtigt. Die zweite Faltung muss damit eine höhere Hürde bzw. einen höheren Schwellwert überwinden, um durch die Vergleichsvorrichtung mit der Bewertung "Schwellwert überschritten" versehen werden zu können. Ein sehr kurz zurückliegendes Störsignal oder Rauschsignal hat damit eine geringere Chance, eine Auswertung zu verfälschen, sodass eine noch sichere Erkennung einer Okklusion oder eines Okklusionsdurchbruches möglich ist.

Bevorzugt weist die Steuerungsmodulvorrichtung zusätzlich auf:
- eine dritte Faltungsfunktionsvorrichtung, welche eingerichtet ist, an ihrem Eingang ein an einer Aspirationsfluidleitung erfasstes zweites Messsignal von einer zweiten Messvorrichtung zu empfangen, und an ihrem Ausgang einen mittels einer dritten Faltungsfunktion gefalteten Betrag einer zeitlichen Ableitung des zweiten Messsignals auszugeben, wobei die dritte Faltungsfunktion eine dritte Zeitdauer besitzt,
- eine dritte Schwellwertvorrichtung, mit welcher sich ein dritter Schwellwert für die zeitliche Ableitung des zweiten Messsignals einrichten lässt,
- eine dritte Vergleichsvorrichtung, welche eingerichtet ist, den von der dritten Faltungsfunktionsvorrichtung ausgegebenen Betrag der zeitlichen Ableitung des zweiten Messsignals zu empfangen und den dritten Schwellwert zu empfangen und nach Ablauf eines vorbestimmten dritten Zeitpunktes den ausgegebenen Betrag der zeitlichen Ableitung des zweiten Messsignals und den dritten Schwellwert miteinander zu vergleichen und als drittes Vergleichsergebnis auszugeben,
- eine vierte Faltungsfunktionsvorrichtung, welche eingerichtet ist, an ihrem Eingang das an der Aspirationsfluidleitung erfasste zweite Messsignal zu empfangen, und an ihrem Ausgang einen mittels einer vierten Faltungsfunktion gefalteten Betrag einer zeitlichen Ableitung des zweiten Messsignals auszugeben, wobei die vierte Faltungsfunktion eine vierte Zeitdauer besitzt, welche kürzer als die dritte Zeitdauer ist,
- eine vierte Schwellwertvorrichtung, mit welcher sich ein vierter Schwellwert für die zeitliche Ableitung des zweiten Messsignals einrichten lässt,
- eine vierte Vergleichsvorrichtung, welche eingerichtet ist, den von der vierten Faltungsfunktionsvorrichtung ausgegebenen Betrag der zeitlichen Ableitung des zweiten Messsignals zu empfangen und den vierten Schwellwert zu empfangen und nach Ablauf eines vorbestimmten vierten Zeitpunktes den ausgegebenen Betrag der zeitlichen Ableitung des zweiten Messsignals und den vierten Schwellwert miteinander zu vergleichen und als viertes Vergleichsergebnis auszugeben,
- eine zweite Auswertevorrichtung, welche eingerichtet ist, das dritte Vergleichsergebnis und das vierte Vergleichsergebnis zu empfangen und auszuwerten und ein zweites Auswertesignal auszugeben,
wobei die Ansteuerungsvorrichtung eingerichtet ist, das zweite Auswertesignal zu empfangen und in Abhängigkeit von dem ersten Auswertesignal und dem zweiten Auswertesignal ein Steuersignal zur Steuerung eines Parameters einer ophthalmochirurgischen Einrichtung bereitzustellen.

Somit kann ein zweites Messsignal mitberücksichtigt werden, welches an einer Aspirationsfluidleitung erfasst wird. Dies ist vorteilhaft, da durch ein erstes Auswertesignal und ein zweites Auswertesignal eine Redundanz erreicht wird, die eine noch sichere Detektion einer Okklusion oder eines Okklusionsdurchbruches ermöglicht.

Gemäß einer Ausführungsform der Erfindung ist das erste Messsignal oder das zweite Messsignal ein Wegsignal eines Wegsensors zur Bestimmung eines Fluidpegels oder zur Bestimmung einer Membranposition. Alternativ dazu ist das erste Messsignal oder das zweite Messsignal ein Volumensignal eines Sensors zur Bestimmung eines Volumens einer Fluidkammer. Dies ist vorteilhaft, da somit die Steuerungsmodulvorrichtung bei einer Membranpumpe eingesetzt werden kann.

Ferner kann die dritte Faltungsfunktion und die vierte Faltungsfunktion punktsymmetrisch zu ihrem jeweiligen Koordinatenursprung sein. Dies ist vorteilhaft, da es somit nach der Faltung zu keiner Verschiebung des Absolutbetrages des ausgegebenen Wertes oder Bevorzugung einzelner Messwerte des Messsignals kommt. Ferner ist eine Punktsymmetrie vorteilhaft für die Genauigkeit der ermittelten Ableitung des ersten Messsignals.

Bevorzugt ist der Betrag des vierten Schwellwertes verschieden von dem Betrag des dritten Schwellwertes. Besonders bevorzugt ist der Betrag des vierten Schwellwertes höher als der Betrag des dritten Schwellwertes. Dadurch wird vermieden, dass ein kurz zurückliegendes Störsignal oder Rauschsignal die Auswertung verfälscht.

Wenn die erste Faltungsfunktion gleich der dritten Faltungsfunktion ist und die zweite Faltungsfunktion gleich der vierten Faltungsfunktion ist, können die Ergebnisse der Auswertevorrichtungen sehr gut miteinander verglichen werden, sodass eine noch zuverlässigere Aussage über das Auftreten einer Okklusion oder eines Okklusionsdurchbruches möglich ist. Dies wird noch weiter verbessert, wenn der erste Schwellwert gleich dem dritten Schwellwert ist und der zweite Schwellwert gleich dem vierten Schwellwert ist.

Das ophthalmochirurgische System weist eine ophthalmochirurgische Steuerungsmodulvorrichtung wie vorstehend beschrieben auf. Zusätzlich umfasst es noch eine ophthalmochirurgische Einrichtung mit einer Irrigationsfluidleitung, einer ersten Fluidpumpe für Irrigation, einer Aspirationsfluidleitung und einer zweiten Fluidpumpe für Aspiration.

Weitere Vorteile und Merkmale der Erfindung werden mit Bezug auf die nachfolgenden Zeichnungen erklärt, in welchen zeigen:
- Fig. 1: eine schematische Darstellung eines ophthalmochirurgischen Systems mit einer ophthalmochirurgischen Steuerungsmodulvorrichtung gemäß einer bevorzugten Ausführungsform der Erfindung;
- Fig. 2: eine schematische Darstellung mit Diagrammen von einem jeweiligen Signalverlauf einer ersten Messvorrichtung und einer zweiten Messvorrichtung und zugehörige Auswertungen der Signalverläufe;
- Fig. 3: eine schematische Darstellung mit Diagrammen von einem jeweiligen Signalverlauf einer ersten Messvorrichtung, wenn 15 Faltungsfunktionen vorgesehen sind; und
- Fig. 4: einen Graphen, bei dem die jeweilige Zeitdauer einer Faltungsfunktion über die Anzahl der Faltungsfunktionen aufgetragen ist.

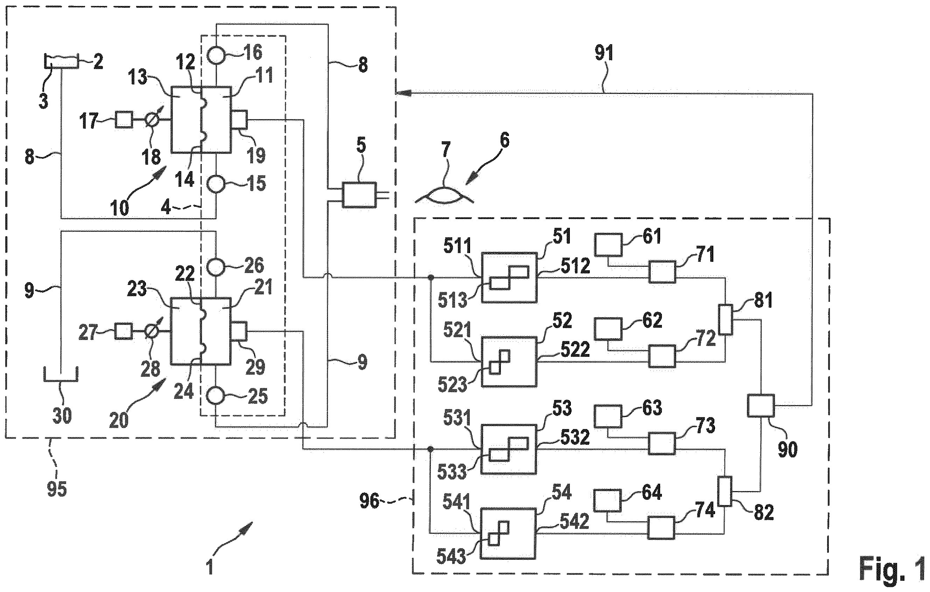

Fig. 1 zeigt eine schematische Darstellung einer Ausführungsform des erfindungsgemäßen ophthalmochirurgischen Systems 1. Das System 1 umfasst eine ophthalmochirurgische Steuerungsmodulvorrichtung 96 und eine ophthalmochirurgische Einrichtung 95.

Die ophthalmochirurgische Einrichtung 95 weist eine Irrigationsfluidleitung 8 auf, die von einem Irrigationsfluidbehälter 2, welcher mit Irrigationsfluid 3 befüllt sein kann, zu einer Kassette 4 verläuft. Die Kassette 4 dient zur Steuerung des Irrigationsfluides 3 zu einem chirurgischen Instrument 5 für ein zu behandelndes Auge 6. Das chirurgische Instrument 5 ist geeignet, eine Linse 7 des Auges 6 zum Beispiel durch Phakoemulsifikation zu zerkleinern, wobei die entstehenden Linsenpartikel und das Irrigationsfluid 3 abgesaugt werden können. Zusätzlich ist in der ophthalmochirurgischen Einrichtung 95 eine erste Fluidpumpe 10 enthalten, welche eine erste Pumpkammer 11 mit einem ersten Volumen und eine mit einem ersten elastischen Trennelement 12 davon getrennte erste Antriebskammer 13 mit einem zweiten Volumen aufweist. Das erste elastische Trennelement 12 ist an seinem Rand 14 fest in der ersten Fluidpumpe 10 montiert.

Das Irrigationsfluid 3 ist mittels der Irrigationsfluidleitung 8 der ersten Pumpkammer 11 zuführbar. Das Irrigationsfluid 3 kann zu einem ersten Einlassventil 15 strömen, bei geöffneter Stellung des Einlassventils 15 in die erste Pumpkammer 11 gelangen und bei einem geöffneten ersten Auslassventil 16 die erste Pumpkammer 11 wieder verlassen. Der benachbart zur ersten Pumpkammer 11 angeordneten ersten Antriebskammer 13 kann ein erstes Antriebsfluid 17 mittels eines Proportionalventils 18 zugeführt werden. In Abhängigkeit von einem Differenzdruck zwischen dem ersten Antriebsfluid 17 in der ersten Antriebskammer 13 und dem Irrigationsfluid 3 in der ersten Pumpkammer 11 kommt es zu einer elastischen Verformung des ersten elastischen Trennelementes 12. Ist der Druck in der ersten Antriebskammer 13 größer als der Druck in der ersten Pumpkammer 11, verkleinert sich das erste Volumen der ersten Pumpkammer 11, wobei sich gleichzeitig das zweite Volumen der ersten Antriebskammer 13 vergrößert. Wenn das Einlassventil 15 geschlossen und das Auslassventil 16 geöffnet ist, kann somit Irrigationsfluid 3 aus der ersten Pumpkammer 11 ausgeleitet werden. Die Position des ersten elastischen Trennelementes 12 kann mittels einer ersten Messvorrichtung 19, zum Beispiel eines ersten Sensors, erfasst werden, welche an der Irrigationsfluidleitung 8, zum Beispiel am Rand der ersten Pumpkammer 11, angeordnet ist. Die Irrigationsfluidleitung 8 wird hier als eine Leitung oder ein Strömungspfad verstanden, durch welchen Irrigationsfluid strömt. Bevorzugt handelt es sich bei der ersten Messvorrichtung 19 um einen induktiven oder kapazitiven Wegsensor.

Während des Zerkleinerns der Linse 7 durch das chirurgische Instrument 5 werden die Partikel zusammen mit dem zugeführten Irrigationsfluid abgesaugt. Dies erfolgt mittels einer zweiten Fluidpumpe 20. Die Linsenpartikel und das beim Zerkleinern der Linse 7 verunreinigte Irrigationsfluid, welches zusammen dann als Aspirationsfluid bezeichnet wird, gelangen entlang einer Aspirationsfluidleitung 9 bei einem geöffneten zweiten Einlassventil 25 zu der zweiten Fluidpumpe 20.

Die zweite Fluidpumpe 20 weist eine zweite Pumpkammer 21 und eine dazu benachbart angeordnete zweite Antriebskammer 23 auf, welche mittels eines zweiten elastischen Trennelementes 22 voneinander getrennt sind. Das zweite elastische Trennelement 22 ist an seinem Rand 24 fest mit der zweiten Fluidpumpe 20 verbunden. Ein zweites Antriebsfluid 27 kann mittels eines Proportionalventils 28 zu der zweiten Antriebskammer 23 geleitet werden. Strömt bei geöffnetem Einlassventil 25 das Aspirationsfluid in die zweite Pumpkammer 21 ein, kann dieses aus der zweiten Pumpkammer 20 aufgrund eines entsprechend hohen Druckes in der zweiten Antriebskammer 23 und dadurch elastisch verformten zweiten Trennelementes 22 heraus befördert werden, wenn das zweite Einlassventil 25 geschlossen und ein zweites Auslassventil 26 geöffnet ist. Die Position des zweiten elastischen Trennelementes 22 kann mittels einer zweiten Messvorrichtung 29, zum Beispiel eines zweiten Sensors, erfasst werden, welche an der Aspirationsfluidleitung, zum Beispiel am Rand der zweiten Pumpkammer 21, angeordnet ist. Das ausgepumpte Aspirationsfluid gelangt entlang der Aspirationsfluidleitung 9 zu einem Aspirationsfluid-Sammelbehälter 30. Die Aspirationsfluidleitung 9 wird hier als eine Leitung oder ein Strömungspfad verstanden, durch welchen Aspirationsfluid strömt. Bevorzugt handelt es sich bei der zweiten Messvorrichtung 29 um einen induktiven oder kapazitiven Wegsensor.

Von der ersten Messvorrichtung 19 kann ein erstes Messsignal der ophthalmochirurgischen Steuerungsmodulvorrichtung 96 zugeführt werden. Das erste Messsignal gelangt dazu an einen Eingang 511 einer ersten Faltungsfunktionsvorrichtung 51. Die erste Faltungsfunktionsvorrichtung 51 ist geeignet, aus Messwerten der ersten Messvorrichtung 19 ein gefaltetes Signal zu erzeugen. Gemäß der Erfindung erzeugt die erste Faltungsfunktionsvorrichtung 51 einen mittels einer ersten Faltungsfunktion 513 gefalteten Betrag einer zeitlichen Ableitung des ersten Messsignals, welches an einem Ausgang 512 der ersten Faltungsfunktionsvorrichtung 51 ausgegeben wird. Die erste Faltungsfunktion 513 weist dabei eine erste Zeitdauer t1 auf.

Die ophthalmochirurgische Steuerungsmodulvorrichtung 96 weist ferner eine erste Schwellwertvorrichtung 61 auf, mit welcher sich ein erster Schwellwert S1 für den Betrag der zeitlichen Ableitung des ersten Messsignals einrichten lässt. Der von der ersten Faltungsfunktionsvorrichtung 51 ausgegebene Betrag der zeitlichen Ableitung des ersten Messsignals wird dann wie auch der erste Schwellwert S1 einer ersten Vergleichsvorrichtung 71 zugeführt und dort nach einem vorbestimmten ersten Zeitpunkt miteinander verglichen. Dabei kann festgestellt werden, dass der ausgegebene Betrag der zeitlichen Ableitung des ersten Messsignals den Schwellwert S1 überschreitet oder nicht überschreitet. Dieses Ergebnis gilt als ein erstes Vergleichsergebnis.

Das erste Messsignal von der ersten Messvorrichtung gelangt zusätzlich an einen Eingang 521 einer zweiten Faltungsfunktionsvorrichtung 52. Die zweite Faltungsfunktionsvorrichtung 52 ist wie die erste Faltungsfunktionsvorrichtung 51 geeignet, aus Messwerten der ersten Messvorrichtung 19 ein gefaltetes Signal zu erzeugen. Gemäß der Erfindung erzeugt die zweite Faltungsfunktionsvorrichtung 52 einen mittels einer zweiten Faltungsfunktion 523 gefalteten Betrag einer zeitlichen Ableitung des ersten Messsignals, welches an einem Ausgang 522 der zweiten Faltungsfunktionsvorrichtung 52 ausgegeben wird. Die zweite Faltungsfunktion 523 weist dabei eine zweite Zeitdauer t2 auf, welche kürzer als die erste Zeitdauer t1 ist.

Die ophthalmochirurgische Steuerungsmodulvorrichtung 96 weist eine zweite Schwellwertvorrichtung 62 auf, mit welcher sich ein zweiter Schwellwert S2 für die zeitliche Ableitung des ersten Messsignals einrichten lässt. Der von der zweiten Faltungsfunktionsvorrichtung 52 ausgegebene Betrag der zeitlichen Ableitung des ersten Messsignals wird dann einer zweiten Vergleichsvorrichtung 72 zugeführt. Diese zweite Vergleichsvorrichtung 72 empfängt zusätzlich den zweiten Schwellwert S2. in der zweiten Vergleichsvorrichtung werden der Betrag der zeitlichen Ableitung des ersten Messsignals und der zweite Schwellwert S2 nach einem vorbestimmten zweiten Zeitpunkt miteinander verglichen. Gemäß einer Ausführungsform ist der zweite Zeitpunkt gleich dem ersten Zeitpunkt. Dabei kann festgestellt werden, dass der ausgegebene Betrag der zeitlichen Ableitung des ersten Messsignals den Schwellwert S2 überschreitet oder nicht überschreitet. Dieses Ergebnis gilt als ein zweites Vergleichsergebnis.

Die ophthalmochirurgische Steuerungsmodulvorrichtung 96 weist zusätzlich eine erste Auswertevorrichtung 81 auf, welche eingerichtet ist, das erste Vergleichsergebnis und das zweite Vergleichsergebnis zu empfangen und auszuwerten und ein erstes Auswertesignal auszugeben. Wenn zum Beispiel beide Schwellwerte S1 und S2 von den ausgegebenen Beträgen der zeitlichen Ableitung des ersten Messsignals überschritten werden, kann daraus gefolgert werden, dass der jeweilige Messwert des Wegsignals der ersten Messvorrichtung 19 mit zunehmendem Zeitablauf mit einer entsprechenden Geschwindigkeit zugenommen hat. Das Überschreiten der Schwellwerte S1 und S2 bedeutet, dass eine Mindestgeschwindigkeit erreicht wurde. Wenn bis dahin noch keine Okklusion vorlag, bedeutet dies, dass in der Irrigationsfluidleitung 8 ein zu erwartender üblicher Volumenstrom des Irrigationsfluides 3 vorliegt.

Wenn nach einem Überschreiten der Schwellwerte S1 und S2 jedoch beide Schwellwerte S1 und S2 von den ausgegebenen Beträgen der zeitlichen Ableitung des ersten Messsignals unterschritten werden, kann daraus gefolgert werden, dass der jeweilige Messwert des Wegsignals der ersten Messvorrichtung 19 mit zunehmendem Zeitablauf mit einer entsprechenden Geschwindigkeit abgenommen hat. Das Unterschreiten der Schwellwerte S1 und S2 bedeutet, dass eine Mindestgeschwindigkeit nicht mehr beibehalten wurde. Mittels der Auswertevorrichtung kann damit erkannt werden, dass sich der Volumenstrom in der Irrigationsfluidleitung geändert hat, und zwar von einem höheren Wert zu einem niedrigeren Wert abgenommen hat. Die Beträge sind unterhalb der Schwellwerte S1 und S2. Dies bedeutet, dass die Strömung in der Irrigationsfluidleitung so niedrig ist, dass dieser Zustand als eine Okklusion bezeichnet werden kann.

Die Auswertung, ob eine Okklusion vorliegt oder nicht, wird dann als erstes Auswertesignal einer Ansteuerungsvorrichtung 90 zugeführt. Die Ansteuerungsvorrichtung 90 kann dann ein Steuersignal 91 zur Steuerung eines Parameters der ophthalmochirurgischen Einrichtung 95 bereitstellen. Dazu kann zum Beispiel die Energiezufuhr an das Handstück 5 unterbrochen werden. Es kann zusätzlich oder alternativ dazu die erste Fluidpumpe 10 und/oder die zweite Fluidpumpe 20 abgeschaltet oder in ihrer Ansteuerung variiert werden, sodass ein unterschiedlicher Volumenstrom und/oder Druck in der zugehörigen Irrigationsfluidleitung 8 oder Aspirationsfluidleitung 9 erreichbar sind.

Wenn nur der zweite Schwellwert S2 unterschritten wird, jedoch der erste Schwellwert S1 nicht unterschritten wird, kann mittels der ersten Auswertevorrichtung 81 festgestellt werden, dass zwar ein unruhiger Verlauf der zeitlichen Ableitung des ersten Messsignals vorliegt, dies aber noch nicht ausreicht, um eine vollständige Okklusion zu identifizieren. Ein solches Ergebnis könnte derart gedeutet werden, dass eine Teilokklusion vorliegt. Ein zugehöriges Auswertesignal wird dann der Ansteuerungsvorrichtung 90 zugeführt, welche ein geeignetes Steuersignal 91 zu Steuerung eines Parameters der ophthalmochirurgischen Einrichtung 95 bereitstellt.

Die in Fig. 1 dargestellte Ausführungsform einer ophthalmochirurgischen Steuerungsmodulvorrichtung 96 weist zusätzlich eine dritte Faltungsfunktionsvorrichtung 53 auf, welche eingerichtet ist, an ihrem Eingang 531 ein an der Aspirationsfluidleitung 9 erfasstes zweites Messsignal von der zweiten Messvorrichtung 29 zu empfangen, und an ihrem Ausgang 532 einen mittels einer dritten Faltungsfunktion 533 gefalteten Betrag einer zeitlichen Ableitung des zweiten Messsignals auszugeben, wobei die dritte Faltungsfunktion 533 eine dritte Zeitdauer t3 besitzt. Bevorzugt ist die dritte Zeitdauer t3 gleich der ersten Zeitdauer t1.

Mittels einer dritten Schwellwertvorrichtung 63 kann ein dritter Schwellwert S3 für die zeitliche Ableitung des zweiten Messsignals eingerichtet werden. Eine dritte Vergleichsvorrichtung 73 ist eingerichtet, den von der dritten Faltungsfunktionsvorrichtung 53 ausgegebenen Betrag der zeitlichen Ableitung des zweiten Messsignals zu empfangen. Die dritte Vergleichsvorrichtung 73 ist zusätzlich eingerichtet, den dritten Schwellwert S3 zu empfangen. Wenn der von der dritten Faltungsfunktionsvorrichtung 53 ausgegebene Betrag der zeitlichen Ableitung des zweiten Messsignals und der dritte Schwellwert S3 der dritten Vergleichsvorrichtung 73 zugeführt werden, kann diese nach einem vorbestimmten dritten Zeitpunkt den Betrag der zeitlichen Ableitung des zweiten Messsignals und den dritten Schwellwert S3 miteinander vergleichen. Dabei kann festgestellt werden, dass der ausgegebene Betrag der zeitlichen Ableitung des zweiten Messsignals den Schwellwert S3 überschreitet oder nicht überschreitet. Dieses Ergebnis gilt als ein drittes Vergleichsergebnis. Bevorzugt ist der dritte Zeitpunkt gleich dem ersten Zeitpunkt.

Das zweite Messsignal von der zweiten Messvorrichtung gelangt zusätzlich an einen Eingang 541 einer vierten Faltungsfunktionsvorrichtung 54. Die vierte Faltungsfunktionsvorrichtung 54 ist wie die dritte Faltungsfunktionsvorrichtung 53 geeignet, aus Messwerten der zweiten Messvorrichtung 29 ein gefaltetes Signal zu erzeugen. Gemäß der Erfindung erzeugt die vierte Faltungsfunktionsvorrichtung 54 einen mittels einer vierten Faltungsfunktion 543 gefalteten Betrag einer zeitlichen Ableitung des zweiten Messsignals, welches an einem Ausgang 542 der vierten Faltungsfunktionsvorrichtung 54 ausgegeben wird. Die vierte Faltungsfunktion 543 weist dabei eine vierte Zeitdauer t4 auf, welche kürzer als die dritte Zeitdauer t3 ist. Bevorzugt ist die vierte Zeitdauer t4 gleich der zweiten Zeitdauer t2.

Mit einer vierten Schwellwertvorrichtung 64 kann ein vierter Schwellwert S4 eingerichtet werden. Dieser vierte Schwellwert S4 und der von der vierten Faltungsfunktionsvorrichtung 54 ausgegebene Betrag der zeitlichen Ableitung des zweiten Messsignals kann einer vierten Vergleichsvorrichtung 74 zugeführt werden, mit welcher nach einem vorbestimmten vierten Zeitpunkt der ausgegebene Betrag der zeitlichen Ableitung des zweiten Messsignals und der vierte Schwellwert S4 miteinander verglichen werden. Mittels der vierten Vergleichsvorrichtung 74 kann festgestellt werden, ob der ausgegebene Betrag der zeitlichen Ableitung des zweiten Messsignals den Schwellwert S4 überschreitet oder nicht überschreitet. Dieses Ergebnis gilt als ein viertes Vergleichsergebnis. Bevorzugt ist der vierte Zeitpunkt gleich dem zweiten Zeitpunkt.

Eine zweite Auswertevorrichtung 82 ist eingerichtet, das dritte Vergleichsergebnis und das vierte Vergleichsergebnis zu empfangen und auszuwerten. Folgende Situation kann vorliegen: Nach dem Einschalten der ophthalmochirurgischen Einrichtung 95 und somit nach dem Strömen des Irrigationsfluides und Aspirationsfluides trat noch keine Okklusion auf. Das dritte Vergleichsergebnis lautet, dass der Schwellwert S3 überschritten wurde und das vierte Vergleichsergebnis lautet, dass der Schwellwert S4 überschritten wurde. Damit hat die Ableitung des zweiten Messsignals beide Schwellwerte S3 und S4 überschritten, sodass das Messsignal während der untersuchten Zeitdauer nach den vorbestimmten dritten und vierten Zeitpunkten einen entsprechenden Anstieg aufwies. In der Aspirationsfluidleitung 9 war somit ein Volumenstrom vorhanden, der als ausreichend hoch bewertet werden kann. Eine Okklusion liegt in der Aspirationsfluidleitung 9 somit nicht vor.

Wenn daraufhin jedoch festgestellt wird, dass das dritte Vergleichsergebnis lautet, dass der dritte Schwellwert S3 unterschritten wird, und das vierte Vergleichsergebnis lautet, dass der vierte Schwellwert S4 ebenfalls unterschritten wird, weist die Ableitung des zweiten Messsignals jeweils einen Betrag unterhalb der Schwellwerte auf. Dies bedeutet, dass die Bewegung des elastischen Trennelementes 22 nur noch sehr gering ist und offenbar sehr wenig Volumenstrom in der Aspirationsfluidleitung 9 existiert. Eine solche Situation kann derart ausgewertet werden, dass eine Okklusion vorliegt. Die zweite Auswertevorrichtung 82 kann dann ein entsprechendes Auswertesignal an die Ansteuerungsvorrichtung 90 ausgeben. Mittels der Ansteuerungsvorrichtung 90 ist es dann möglich, ein Steuersignal 91 zur Steuerung eines Parameters der ophthalmochirurgischen Einrichtung 95 bereitzustellen.

Wenn nur der Schwellwert 54, jedoch nicht der Schwellwert S3 unterschritten wird, scheint ein unruhiger Signalverlauf mit relativ hohen Schwankungen oder relativ hohem Rauschen vorzuliegen. Dann kann die Auswertevorrichtung 82 zu dem Ergebnis kommen, dass noch keine vollständige Okklusion vorliegt bzw. eine Teilokklusion vorliegt.

Besonders geschickt ist es, wenn die Ansteuerungsvorrichtung 90 das erste Auswertesignal und das zweite Auswertesignal empfängt, da somit eine Redundanz erreichbar ist. Die Ansteuerungsvorrichtung 90 kann so ausgebildet sein, dass nur dann ein Steuersignal 91 an die ophthalmochirurgische Einrichtung 95 abgegeben wird, wenn die erste Auswertevorrichtung 81 und die zweite Auswertevorrichtung 82 zu einem gleichen Auswerteergebnis kommen.

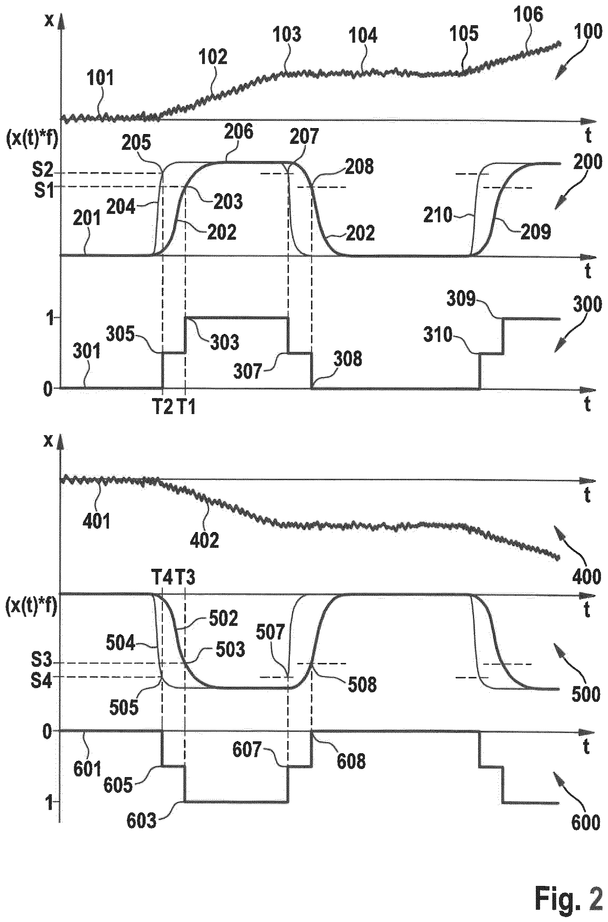

In Fig. 2 sind mehrere schematische Diagramme dargestellt. Das erste Diagramm 100 zeigt einen Verlauf eines Weges x des elastischen Trennelementes 12 der ersten Fluidpumpe 10 in Abhängigkeit von der Zeit t. Dieser Verlauf kann durch die erste Messvorrichtung 19 ermittelt werden. Es wird angenommen, dass sich das elastische Trennelement 12 anfangs in einer ersten Position befindet, siehe 101. Wenn das Antriebsfluid 17 in die Antriebskammer 13 befördert wird, bewegt sich das elastische Trennelement 12 derart, dass Irrigationsfluid 3 aus der Pumpkammer 11 befördert wird. Das elastische Trennelement 12 bewegt sich somit näher an die erste Messvorrichtung 19 heran, siehe 102. Wenn eine Okklusion in der Aspirationsfluidleitung 9 auftritt, siehe 103, kann kein weiteres Fluid mehr in das Auge zugeführt werden, sodass die Position des elastischen Trennelementes 12 sich nicht mehr ändert, siehe 104. Bei einem Durchbruch der Okklusion, siehe 105, kann wieder Irrigationsfluid in der Irrigationsfluidleitung 8 strömen, so dass sich das elastische Trennelement 12 wieder näher an die erste Messvorrichtung 19 heran bewegt, siehe 106. In Diagramm 100 ist erkennbar, dass der Signalverlauf nicht vollständig glatt, sondern mit signifikantem Rauschen vorliegt.

Wenn das Signal der ersten Messvorrichtung 19 der ersten Faltungsfunktionsvorrichtung 51 zugeführt wird und dort mit einer ersten Faltungsfunktionf1, siehe 513, mit einer ersten Zeitdauer t1 so gefaltet wird, dass eine zeitliche Ableitung des ersten Messsignals erfolgt, kann an dem Ausgang 512 ein Signal (*x*(t) * *f*1) abgegriffen werden, welches in Diagramm 200 dargestellt ist. Die Ableitung erreicht anfangs den Wert null, siehe 201, und steigt dann mit einer zu der ersten Faltungsfunktion 513 zugehörigen Zeitkonstante, welche zum Beispiel 0,5 Sekunden beträgt, an. Dieser Kurvenverlauf ist mit 202 bezeichnet. Das erste Messsignal wird bevorzugt gleichzeitig der zweiten Faltungsfunktionsvorrichtung 52 zugeführt, welche das Signal mittels einer zweiten Faltungsfunktion *f*2, siehe 523, mit einer im Vergleich zur ersten Zeitdauer t1 kürzeren Zeitdauer t2, z.B. 50 Millisekunden, faltet, sodass am Ausgang 522 ein Signal (*x*(t) * *f*2) abgegriffen werden kann. Nach Einsetzen der Pumpfunktion der ersten Fluidpumpe 10 erreicht die zeitliche Ableitung des ersten Messsignals einen Verlauf, siehe 204, der eine größere Steigung aufweist als die zeitliche Ableitung, die nach der ersten Faltungsfunktionsvorrichtung 51 erreicht wird.

Die Kurve 202 schneidet einen ersten Schwellwert S1 an der Stelle 203. Die Kurve 204 schneidet einen zweiten Schwellwert S2 an der Stelle 205, wobei der zweite Schwellwert S2 höher als der erste Schwellwert S1 ist. In Diagramm 300 ist aufgetragen, an welchen Zeitpunkten ein Schnittpunkt zwischen der Kurve 202 mit dem ersten Schwellwert S1 und der Kurve 204 mit dem zweiten Schwellwert S2 auftritt. Die Kurve 202 schneidet den ersten Schwellwert S1 nach Ablauf eines vorbestimmten ersten Zeitpunktes. Bei der in Fig. 2 dargestellten Situation schneidet die Kurve 202 den ersten Schwellwert S1 zum Zeitpunkt T1. Die Kurve 204 schneidet den zweiten Schwellwert S2 nach Ablauf eines vorbestimmten zweiten Zeitpunktes. Bei der in Fig. 2 dargestellten Situation schneidet die Kurve 204 den zweiten Schwellwert S2 zum Zeitpunkt T2. Wenn also beide Schwellwerte S1 und S2 überschritten werden, ist eine relativ sichere Aussage möglich, dass in der Irrigationsfluidleitung 8 ein Irrigationsfluid strömt. Diese Aussage kann anhand des Kurvenverlaufs in dem dritten Diagramm 300 getroffen werden. Bei einem Überschreiten eines Schwellwertes kommt es zu einer sprungartigen Erhöhung der Kurve 301. Beim Überschreiten des Schwellwertes S2 ergibt sich ein erster Sprung, siehe 305, beim Überschreiten des ersten Schwellwertes S1 ergibt sich ein zweiter Sprung, siehe 303.

Wenn nur der Schwellwert S2 von der Kurve 204 überschritten wird, nicht jedoch der Schwellwert S1 von der Kurve 202, ist dies ein Anzeichen dafür, dass nach Ablauf des ersten Zeitpunktes der Anstieg der Kurve 202 bereits beendet ist. Dies zeigt auf, dass sich das elastische Trennelement 12 nicht mehr in Richtung zur ersten Messvorrichtung hin bewegt und der Volumenstrom in der Irrigationsfluidleitung unterbrochen ist.

Wenn jedoch ein relativ konstanter Volumenstrom in der Irrigationsfluidleitung 8 vorliegt, erreicht der gefaltete Betrag der Ableitung des ersten Messsignals einen konstant hohen Wert, siehe 206. Wird nun der ersten Faltungsfunktionsvorrichtung 51 ein erstes Messsignal zugeführt, welches nach der Faltung mittels der ersten Faltungsfunktionsvorrichtung 51 den zweiten Schwellwert S2 unterschreitet, siehe 207, und auch den ersten Schwellwert S1 unterschreitet, siehe 208, so sinkt die Kurve 301 wieder sprungartig ab, siehe 307 und 308. Daraus kann erkannt werden, dass ein Volumenstrom des Irrigationsfluides 3 in der Irrigationsfluidleitung 8 abgenommen hat.

Wenn dann die Kurven 202 und 204 wieder ansteigen, siehe 209 und 210, und den ersten Schwellwert S1 und den zweiten Schwellwert S2 überschreiten, lässt sich an dem dritten Diagramm 300 anhand der sprungartigen Erhöhungen, siehe 309 und 310, erkennen, dass eine Okklusion wieder aufgebrochen ist.

Eine noch höhere Sicherheit wird erreicht, wenn eine analoge Auswertung auch für das zweite Messsignal der zweiten Messvorrichtung 29 vorgenommen wird. Im vierten Diagramm 400 ist ein Weg-Zeit-Diagramm *x*(*t*) für die Bewegung des zweiten elastischen Trennelementes 22 dargestellt. Von einer Ausgangsposition 401 bewegt sich das zweite elastische Trennelement 22 immer mehr von der zweiten Messvorrichtung 29 fort, siehe 402. Das zweite Messsignal wird der dritten Faltungsfunktionsvorrichtung 53 zugeführt, welche das Messsignal mittels der dritten Faltungsfunktion *f*3, siehe 533, faltet. Der so erreichte Betrag (*x*(t) * *f*3) für die Ableitung des zweiten Messsignals kann am Ausgang 532 abgegriffen werden und führt zu einer Kurve 502, siehe fünftes Diagramm 500. Das mittels der vierten Faltungsfunktionsvorrichtung 54 bzw. der vierten Faltungsfunktion *f*4, siehe 543, zu (*x*(t) * *f*4) gefaltete Messsignal führt am Ausgang 542 zu einer Ableitung gemäß Kurve 504. Die Kurve 502 schneidet nach Ablauf eines vorbestimmten dritten Zeitpunktes den dritten Schwellwert S3 bei 503. Dieser Schnittpunkt erfolgt zum Zeitpunkt T3. Die Kurve 504 schneidet nach Ablauf eines vierten Zeitpunktes den vierten Schwellwert S4 bei 505. Dieser Schnittpunkt erfolgt zum Zeitpunkt T4. Diese Ereignisse lassen sich aus dem sechsten Diagramm 600 erkennen, siehe 603 und 605. Wenn die Kurven 504 und 503 wieder absinken und einen dritten Schwellwert S3 und einen vierten Schwellwert S4 unterschreiten, ist der Volumenstrom in der Aspirationsfluidleitung 9 offenbar so niedrig, dass auf eine Okklusion geschlossen werden kann. Dies lässt sich aus dem Verlauf der Kurve 601 in dem sechsten Diagramm 600 an den Stellen 607 und 608 erkennen.

Diese Information, dass eine Okklusion vorliegt, konnte bereits durch Auswertung des ersten Messsignals ermöglicht werden. Die Auswertung auch des zweiten Messsignals erlaubt eine Redundanz. Es kann zum Beispiel festgelegt werden, dass eine Okklusion nur dann als festgestellt gilt, wenn dieses Ergebnis durch das erste Messsignal und durch das zweite Messsignal erreicht wird (UND-Verknüpfung gemäß der Boolschen Algebra). Dies erhöht die Zuverlässigkeit in der Auswertung der Signalverläufe.

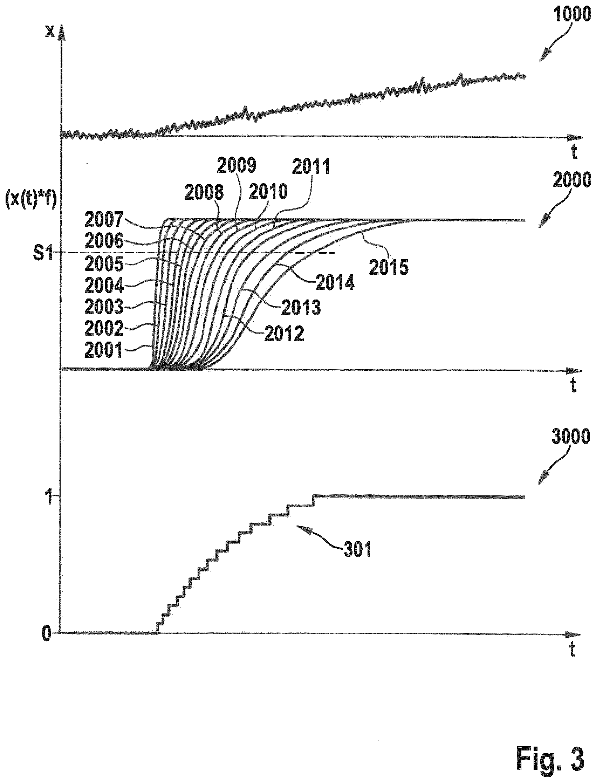

Bevorzugt weist die Steuerungsmodulvorrichtung mehr als zwei Faltungsfunktionsvorrichtungen für die Auswertung des ersten Messsignals auf. Besonders bevorzugt nimmt mit zunehmender Zahl der mit dem Messsignal beaufschlagten Faltungsfunktionen die jeweils zugehörige Zeitdauer einer Faltungsfunktion ab. Wenn zum Beispiel 15 Faltungsfunktionsvorrichtungen vorgesehen sind, welchen das erste Messsignal zugeführt wird, weist die 1. Faltungsfunktionsvorrichtung eine Faltungsfunktion mit einer ersten Zeitdauer auf, welche die längste Zeitdauer im Vergleich zu den nachfolgenden Faltungsfunktionen ist. Die 2. Faltungsfunktionsvorrichtung weist eine Faltungsfunktion mit einer zweiten Zeitdauer auf, welche eine kürzere Zeitdauer als die erste Zeitdauer ist, usw. Dies führt zu 15 Stufensprüngen im Verlauf der Kurve 301, vgl. Fig. 3. Das Diagramm 1000 zeigt einen Verlauf eines Weges des elastischen Trennelementes 12 der ersten Fluidpumpe 10 in Abhängigkeit von der Zeit. Das Diagramm 2000 zeigt den Verlauf der Beträge in Abhängigkeit von der Zeit, welche am Ausgang der jeweiligen Faltungsfunktionsvorrichtungen ausgegeben werden, siehe Kurven 2001 bis 2015. Wird jeweils ein Schwellwert S1 überschritten, wird dies durch die Auswertevorrichtung 81 erkannt, sodass die Kurve 301 fein gestuft verläuft, siehe Diagramm 3000.

Bei der hier gezeigten Ausführungsform wird jeder der von den 15 Faltungsfunktionsvorrichtungen ausgegebene Betrag mit einem identischen Schwellwert S1 verglichen. Gemäß einer weiteren Ausführungsform können die von den 15 Faltungsfunktionsvorrichtungen ausgegebenen Beträge mit jeweils voneinander verschiedenen Schwellwerten S1 bis S15 verglichen werden.

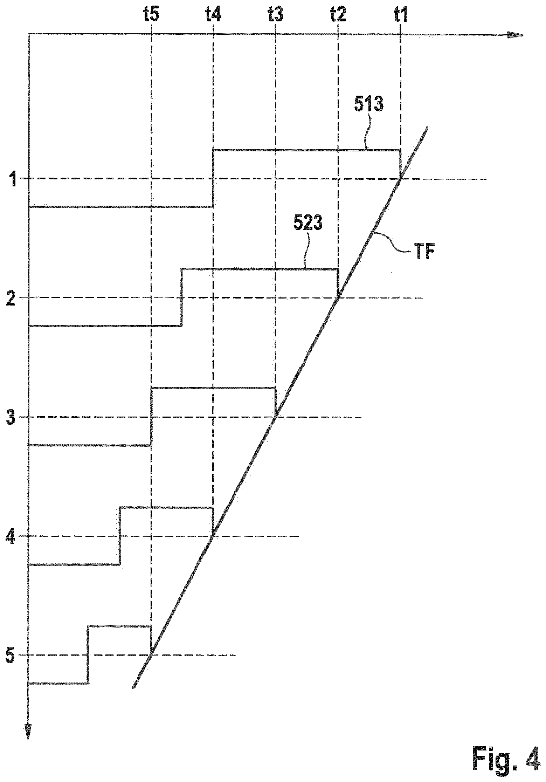

Jede der Faltungsfunktionen weist eine Zeitdauer auf, in der Messwerte eines Messsignals verarbeitet werden. Mit zunehmender Zahl der Faltungsfunktionen, denen ein Messsignal jeweils zugeführt wird, nimmt die diesen Faltungsfunktionen zugehörige Zeitdauer ab. Wenn eine jeweilige Zeitdauer einer Faltungsfunktion über die Anzahl der Faltungsfunktionen aufgetragen wird, kann eine solche Zeitdauerkurve einen linearen, exponentiellen oder logarithmischen Verlauf einnehmen. Fig. 4 zeigt als Beispiel einen linearen Verlauf einer solchen mit TF bezeichneten Kurve, wenn 5 Faltungsfunktionen vorgesehen sind.

## Patentansprüche

1. Ophthalmochirurgische Steuerungsmodulvorrichtung (96), aufweisend:
- eine erste Faltungsfunktionsvorrichtung (51), welche eingerichtet ist, an ihrem Eingang (511) ein an einer Irrigationsfluidleitung (8) erfasstes erstes Messsignal von einer ersten Messvorrichtung (19) zu empfangen, und an ihrem Ausgang (512) einen mittels einer ersten Faltungsfunktion (513) gefalteten Betrag einer zeitlichen Ableitung des ersten Messsignals auszugeben, wobei die erste Faltungsfunktion (513) eine erste Zeitdauer (t1) besitzt,
- eine erste Schwellwertvorrichtung (61), mit welcher sich ein erster Schwellwert (S1) für die zeitliche Ableitung des ersten Messsignals einrichten lässt,
- eine erste Vergleichsvorrichtung (71), welche eingerichtet ist, den von der ersten Faltungsfunktionsvorrichtung (51) ausgegebenen Betrag der zeitlichen Ableitung des ersten Messsignals zu empfangen und den ersten Schwellwert (S1) zu empfangen und nach Ablauf eines vorbestimmten ersten Zeitpunktes den ausgegebenen Betrag der zeitlichen Ableitung des ersten Messsignals und den ersten Schwellwert (S1) miteinander zu vergleichen und ein erstes Vergleichsergebnis auszugeben,
**dadurch gekennzeichnet, dass** die ophthalmochirurgische Steuerungsmodulvorrichtung weiterhin aufweist:
- eine zweite Faltungsfunktionsvorrichtung (52), welche eingerichtet ist, an ihrem Eingang (521) das an der Irrigationsfluidleitung (8) erfasste erste Messsignal zu empfangen, und an ihrem Ausgang (522) einen mittels einer zweiten Faltungsfunktion (523) gefalteten Betrag einer zeitlichen Ableitung des ersten Messsignals auszugeben, wobei die zweite Faltungsfunktion (523) eine zweite Zeitdauer (t2) besitzt, welche kürzer als die erste Zeitdauer (t1) ist,
- eine zweite Schwellwertvorrichtung (62), mit welcher sich ein zweiter Schwellwert (S2) für die zeitliche Ableitung des ersten Messsignals einrichten lässt,
- eine zweite Vergleichsvorrichtung (72), welche eingerichtet ist, den von der zweiten Faltungsfunktionsvorrichtung (52) ausgegebenen Betrag der zeitlichen Ableitung des ersten Messsignals zu empfangen und den zweiten Schwellwert (S2) zu empfangen und nach Ablauf eines vorbestimmten zweiten Zeitpunktes den ausgegebenen Betrag der zeitlichen Ableitung des ersten Messsignals und den zweiten Schwellwert (S2) miteinander zu vergleichen und ein zweites Vergleichsergebnis auszugeben,
- eine erste Auswertevorrichtung (81), welche eingerichtet ist, das erste Vergleichsergebnis und das zweite Vergleichsergebnis zu empfangen und auszuwerten und ein erstes Auswertesignal auszugeben,
- eine Ansteuerungsvorrichtung (90), welche eingerichtet ist, das erste Auswertesignal zu empfangen und in Abhängigkeit von dem ersten Auswertesignal ein Steuersignal (91) zur Steuerung eines Parameters einer ophthalmochirurgischen Einrichtung (95) bereitzustellen.

2. Steuerungsmodulvorrichtung (96) nach Anspruch 1, wobei die erste Faltungsfunktion (513) und die zweite Faltungsfunktion (523) punktsymmetrisch zu ihrem jeweiligen Koordinatenursprung sind.

3. Steuerungsmodulvorrichtung (96) nach einem der Ansprüche 1 oder 2, wobei der Betrag des zweiten Schwellwertes (S2) verschieden von dem Betrag des ersten Schwellwertes (S1) ist.

4. Steuerungsmodulvorrichtung (96) nach einem der vorherigen Ansprüche, aufweisend:
- eine dritte Faltungsfunktionsvorrichtung (53), welche eingerichtet ist, an ihrem Eingang (531) ein an einer Aspirationsfluidleitung (9) erfasstes zweites Messsignal von einer zweiten Messvorrichtung (29) zu empfangen, und an ihrem Ausgang (533) einen mittels einer dritten Faltungsfunktion (533) gefalteten Betrag einer zeitlichen Ableitung des zweiten Messsignals auszugeben, wobei die dritte Faltungsfunktion (533) eine dritte Zeitdauer (t3) besitzt,
- eine dritte Schwellwertvorrichtung (63), mit welcher sich ein dritter Schwellwert (S3) für die zeitliche Ableitung des zweiten Messsignals einrichten lässt,
- eine dritte Vergleichsvorrichtung (73), welche eingerichtet ist, den von der dritten Faltungsfunktionsvorrichtung (53) ausgegebenen Betrag der zeitlichen Ableitung des zweiten Messsignals zu empfangen und den dritten Schwellwert (S3) zu empfangen und nach Ablauf eines vorbestimmten dritten Zeitpunktes den ausgegebenen Betrag der zeitlichen Ableitung des zweiten Messsignals und den dritten Schwellwert (S3) miteinander zu vergleichen und als drittes Vergleichsergebnis auszugeben,
- eine vierte Faltungsfunktionsvorrichtung (54), welche eingerichtet ist, an ihrem Eingang (541) das an der Aspirationsfluidleitung (9) erfasste zweite Messsignal zu empfangen, und an ihrem Ausgang (542) einen mittels einer vierten Faltungsfunktion (543) gefalteten Betrag einer zeitlichen Ableitung des zweiten Messsignals auszugeben, wobei die vierte Faltungsfunktion (543) eine vierte Zeitdauer (t4) besitzt, welche kürzer als die dritte Zeitdauer (t3) ist,
- eine vierte Schwellwertvorrichtung (64), mit welcher sich ein vierter Schwellwert (S4) für die zeitliche Ableitung des zweiten Messsignals einrichten lässt,
- eine vierte Vergleichsvorrichtung (74), welche eingerichtet ist, den von der vierten Faltungsfunktionsvorrichtung (54) ausgegebenen Betrag der zeitlichen Ableitung des zweiten Messsignals zu empfangen und den vierten Schwellwert (S4) zu empfangen und nach Ablauf eines vorbestimmten vierten Zeitpunktes den ausgegebenen Betrag der zeitlichen Ableitung des zweiten Messsignals und den vierten Schwellwert (S4) miteinander zu vergleichen und als viertes Vergleichsergebnis auszugeben,
- eine zweite Auswertevorrichtung (82), welche eingerichtet ist, das dritte Vergleichsergebnis und das vierte Vergleichsergebnis zu empfangen und auszuwerten und ein zweites Auswertesignal auszugeben,
wobei die Ansteuerungsvorrichtung (90) eingerichtet ist, das zweite Auswertesignal zu empfangen und in Abhängigkeit von dem ersten Auswertesignal und dem zweiten Auswertesignal ein Steuersignal (91) zur Steuerung eines Parameters einer ophthalmochirurgischen Einrichtung (95) bereitzustellen.

5. Steuerungsmodulvorrichtung (96) nach einem der vorherigen Ansprüche, wobei das erste Messsignal oder das zweite Messsignal ein Wegsignal eines Wegsensors zur Bestimmung eines Fluidpegels oder zur Bestimmung einer Membranposition ist oder wobei das erste Messsignal oder das zweite Messsignal ein Volumensignal eines Sensors zur Bestimmung eines Volumens einer Fluidkammer ist.

6. Steuerungsmodulvorrichtung (96) nach Anspruch 4 oder 5, wobei die dritte Faltungsfunktion (533) und die vierte Faltungsfunktion (543) punktsymmetrisch zu ihrem jeweiligen Koordinatenursprung sind.

7. Steuerungsmodulvorrichtung (96) nach einem der Ansprüche 4 bis 6, wobei der Betrag des vierten Schwellwertes (S4) verschieden von dem Betrag des dritten Schwellwertes (S3) ist.

8. Steuerungsmodulvorrichtung (96) nach einem der Ansprüche 4 bis 7, wobei die erste Faltungsfunktion (513) gleich der dritten Faltungsfunktion (533) ist und die zweite Faltungsfunktion (523) gleich der vierten Faltungsfunktion (543) ist.

9. Steuerungsmodulvorrichtung (96) nach einem der Ansprüche 4 bis 8, wobei der erste Schwellwert (S1) gleich dem dritten Schwellwert (S3) ist und der zweite Schwellwert (S2) gleich dem vierten Schwellwert (S4) ist.

10. Ophthalmochirurgisches System (1), welches eine ophthalmochirurgische Steuerungsmodulvorrichtung (96) gemäß einem der vorherigen Ansprüche und eine ophthalmochirurgische Einrichtung (95) mit einer Irrigationsfluidleitung (8), einer ersten Fluidpumpe (10) für Irrigation, einer Aspirationsfluidleitung (9) und einer zweiten Fluidpumpe (20) für Aspiration aufweist.

## Claims

1. Ophthalmosurgical control module device (96), having:
- a first convolution function device (51), which is configured to receive, at its input (511), a first measurement signal of a first measuring device (19) detected on an irrigation fluid line (8) and, at its output (512), to output a quantity, convolved by means of a first convolution function (513), of a time derivative of the first measurement signal, wherein the first convolution function (513) has a first time duration (t1),
- a first threshold value device (61), with which a first threshold value (S1) for the time derivative of the first measurement signal can be established,
- a first comparison device (71), which is configured to receive the quantity, output by the first convolution function device (51), of the time derivative of the first measurement signal and to receive the first threshold value (S1) and, after expiration of a predetermined first time point, to compare the output quantity of the time derivative of the first measurement signal and the first threshold value (S1) with each other and output a first comparison result,
**characterized in that** the ophthalmosurgical control module device further has:
- a second convolution function device (52), which is configured to receive, at its input (521), the first measurement signal detected on the irrigation fluid line (8) and, at its output (522), to output a quantity, convolved by means of a second convolution function (523), of a time derivative of the first measurement signal, wherein the second convolution function (523) has a second time duration (t2), which is shorter than the first time duration (t1),
- a second threshold value device (62), with which a second threshold value (S2) for the time derivative of the first measurement signal can be established,
- a second comparison device (72), which is configured to receive the quantity, output by the second convolution function device (52), of the time derivative of the first measurement signal and to receive the second threshold value (S2) and, after expiration of a predetermined second time point, to compare the output quantity of the time derivative of the first measurement signal and the second threshold value (S2) with each other and output a second comparison result,
- a first evaluation device (81), which is configured to receive and evaluate the first comparison result and the second comparison result and output a first evaluation signal,
- an activation device (90), which is configured to receive the first evaluation signal and, in accordance with the first evaluation signal, to make available a control signal (91) for controlling a parameter of an ophthalmosurgical appliance (95).

2. Control module device (96) according to Claim 1, wherein the first convolution function (513) and the second convolution function (523) are point-symmetrical in relation to their respective coordinate origin.

3. Control module device (96) according to either of Claims 1 and 2, wherein the quantity of the second threshold value (S2) is different than the quantity of the first threshold value (S1).

4. Control module device (96) according to one of the preceding claims, having:
- a third convolution function device (53), which is configured to receive, at its input (531), a second measurement signal of a second measuring device (29) detected on an aspiration fluid line (9) and, at its output (533), to output a quantity, convolved by means of a third convolution function (533), of a time derivative of the second measurement signal, wherein the third convolution function (533) has a third time duration (t3),
- a third threshold value device (63), with which a third threshold value (S3) for the time derivative of the second measurement signal can be established,
- a third comparison device (73), which is configured to receive the quantity, output by the third convolution function device (53), of the time derivative of the second measurement signal and to receive the third threshold value (S3) and, after expiration of a predetermined third time point, to compare the output quantity of the time derivative of the second measurement signal and the third threshold value (S3) with each other and output it as third comparison result,
- a fourth convolution function device (54), which is configured to receive, at its input (541), the second measurement signal detected on the aspiration fluid line (9) and, at its output (542), to output a quantity, convolved by means of a fourth convolution function (543), of a time derivative of the second measurement signal, wherein the fourth convolution function (543) has a fourth time duration (t4), which is shorter than the third time duration (t3),
- a fourth threshold value device (64), with which a fourth threshold value (S4) for the time derivative of the second measurement signal can be established,
- a fourth comparison device (74), which is configured to receive the quantity, output by the fourth convolution function device (54), of the time derivative of the second measurement signal and to receive the fourth threshold value (S4) and, after expiration of a predetermined fourth time point, to compare the output quantity of the time derivative of the second measurement signal and the fourth threshold value (S4) with each other and output it as fourth comparison result,
- a second evaluation device (82), which is configured to receive and evaluate the third comparison result and the fourth comparison result and output a second evaluation signal,
wherein the activation device (90) is configured to receive the second evaluation signal and, in accordance with the first evaluation signal and the second evaluation signal, to make available a control signal (91) for controlling a parameter of an ophthalmosurgical appliance (95).

5. Control module device (96) according to one of the preceding claims, wherein the first measurement signal or the second measurement signal is a displacement signal of a displacement sensor for determining a fluid level or for determining a membrane position, or wherein the first measurement signal or the second measurement signal is a volume signal of a sensor for determining a volume of a fluid chamber.

6. Control module device (96) according to Claim 4 or 5, wherein the third convolution function (533) and the fourth convolution function (543) are point-symmetrical in relation to their respective coordinate origin.

7. Control module device (96) according to one of Claims 4 to 6, wherein the quantity of the fourth threshold value (S4) is different than the quantity of the third threshold value (S3).

8. Control module device (96) according to one of Claims 4 to 7, wherein the first convolution function (513) is equal to the third convolution function (533), and the second convolution function (523) is equal to the fourth convolution function (543).

9. Control module device (96) according to one of Claims 4 to 8, wherein the first threshold value (S1) is equal to the third threshold value (S3), and the second threshold value (S2) is equal to the fourth threshold value (S4).

10. Ophthalmosurgical system (1), which has an ophthalmosurgical control module device (96) according to one of the preceding claims and an ophthalmosurgical appliance (95) with an irrigation fluid line (8), a first fluid pump (10) for irrigation, an aspiration fluid line (9), and a second fluid pump (20) for aspiration.

## Revendications

1. Dispositif (96) formant module de commande de chirurgie ophtalmique, comprenant :
- un premier dispositif (51) à fonction de convolution qui est conçu pour recevoir à son entrée (511) un premier signal de mesure détecté sur une conduite (8) de fluide d'irrigation, provenant d'un premier dispositif de mesure (19), et pour délivrer à sa sortie (512) une valeur d'une dérivée par rapport au temps du premier signal de mesure, qui a été convoluée au moyen d'une première fonction de convolution (513), la première fonction de convolution (513) ayant une première durée (t1),
- un premier dispositif de seuil (61) permettant d'établir une première valeur de seuil (S1) pour la dérivée par rapport au temps du premier signal de mesure,
- un premier dispositif de comparaison (71) qui est conçu pour recevoir la valeur de la dérivée par rapport au temps du premier signal de mesure émis par le premier dispositif (51) à fonction de convolution et pour recevoir la première valeur de seuil (S1) et, après l'écoulement d'un premier temps prédéterminé, pour comparer entre elles la valeur émise de la dérivée par rapport au temps du premier signal de mesure et la première valeur de seuil (S1), et pour délivrer un premier résultat de comparaison,
**caractérisé en ce que** le dispositif formant module de commande de chirurgie ophtalmique comprend en outre :
- un deuxième dispositif (52) à fonction de convolution qui est conçu pour recevoir à son entrée (521) le premier signal de mesure détecté sur la conduite (8) de fluide d'irrigation et pour délivrer à sa sortie (522) une valeur d'une dérivée par rapport au temps du premier signal de mesure, qui a été convoluée au moyen d'une deuxième fonction de convolution (523), la deuxième fonction de convolution (523) ayant une deuxième durée (t2) qui est plus courte que la première durée (t1),
- un deuxième dispositif de seuil (62) permettant d'établir une deuxième valeur de seuil (S2) pour la dérivée par rapport au temps du premier signal de mesure,
- un deuxième dispositif de comparaison (72) qui est conçu pour recevoir la valeur de la dérivée par rapport au temps du premier signal de mesure émise par le deuxième dispositif (52) à fonction de convolution et pour recevoir la deuxième valeur de seuil (S2) et, après l'écoulement d'un deuxième temps prédéterminé, pour comparer entre elles la valeur émise de la dérivée par rapport au temps du premier signal de mesure et la deuxième valeur de seuil (S2), et pour délivrer un deuxième résultat de comparaison,
- un premier dispositif d'évaluation (81) qui est conçu pour recevoir et évaluer le premier résultat de comparaison et le deuxième résultat de comparaison, et pour délivrer un premier signal d'évaluation,
- un dispositif de commande (90) qui est conçu pour recevoir le premier signal d'évaluation et pour fournir, en fonction du premier signal d'évaluation, un signal de commande (91) pour commander un paramètre d'un dispositif (95) de chirurgie ophtalmique.

2. Dispositif (96) formant module de commande selon la revendication 1, dans lequel la première fonction de convolution (513) et la deuxième fonction de convolution (523) sont à symétrie ponctuelle par rapport à l'origine de leurs coordonnées respectives.

3. Dispositif (96) formant module de commande selon l'une des revendications 1 ou 2, dans lequel la valeur de la deuxième valeur de seuil (S2) est différente de la valeur de la première valeur de seuil (S1).

4. Dispositif (96) formant module de commande selon l'une des revendications précédentes, comprenant :
- un troisième dispositif (53) à fonction de convolution conçu pour recevoir à son entrée (531), en provenance d'un deuxième dispositif de mesure (29), un deuxième signal de mesure détecté sur une conduite (9) de fluide d'aspiration, et pour délivrer à sa sortie (533) une valeur d'une dérivée par rapport au temps du deuxième signal de mesure, qui a été convoluée au moyen d'une troisième fonction de convolution (533), la troisième fonction de convolution (533) ayant une troisième durée (t3),
- un troisième dispositif de seuil (63) permettant d'établir une troisième valeur de seuil (S3) pour la dérivée par rapport au temps du deuxième signal de mesure,
- un troisième dispositif de comparaison (73), qui est conçu pour recevoir la valeur de la dérivée par rapport au temps du deuxième signal de mesure émise par le troisième dispositif (53) à fonction de convolution et pour recevoir la troisième valeur de seuil (S3) et, après l'écoulement d'un troisième temps prédéterminé, pour comparer entre elles la valeur émise de la dérivée par rapport au temps du deuxième signal de mesure et la troisième valeur de seuil (S3), et pour les délivrer en tant que troisième résultat de comparaison,
- un quatrième dispositif (54) à fonction de convolution qui est conçu pour recevoir à son entrée (541) le deuxième signal de mesure détecté sur la conduite (9) de fluide d'aspiration et pour délivrer à sa sortie (542) une valeur d'une dérivée par rapport au temps du deuxième signal de mesure, qui a été convoluée au moyen d'une quatrième fonction de convolution (543), la quatrième fonction de convolution (543) ayant une quatrième durée (t4) qui est plus courte que la troisième durée (t3),
- un quatrième dispositif de seuil (64) permettant d'établir une quatrième valeur de seuil (S4) pour la dérivée par rapport au temps du deuxième signal de mesure,
- un quatrième dispositif de comparaison (74) qui est conçu pour recevoir la valeur de la dérivée par rapport au temps du deuxième signal de mesure émise par le quatrième dispositif (54) à fonction de convolution et pour recevoir la quatrième valeur de seuil (S4) et, après l'écoulement d'un quatrième temps prédéterminé, pour comparer entre elles la valeur de la dérivée par rapport au temps du deuxième signal de mesure et la quatrième valeur de seuil (S4), et pour les délivrer en tant que quatrième résultat de comparaison,
- un deuxième dispositif d'évaluation (82), qui est conçu pour recevoir et évaluer le troisième résultat de comparaison et le quatrième résultat de comparaison et pour délivrer un deuxième signal d'évaluation,
le dispositif de commande (90) étant conçu pour recevoir le deuxième signal d'évaluation et pour fournir, en fonction du premier signal d'évaluation et du deuxième signal d'évaluation, un signal de commande (91) pour commander un paramètre d'un dispositif (95) de chirurgie ophtalmique.

5. Dispositif (96) formant module de commande selon l'une des revendications précédentes, dans lequel le premier signal de mesure ou le deuxième signal de mesure est un signal de déplacement d'un capteur de déplacement pour déterminer un niveau de fluide ou pour déterminer une position de membrane, ou dans lequel le premier signal de mesure ou le deuxième signal de mesure est un signal de volume d'un capteur pour déterminer un volume d'une chambre à fluide.

6. Dispositif (96) formant module de commande selon la revendication 4 ou 5, dans lequel la troisième fonction de convolution (533) et la quatrième fonction de convolution (543) sont à symétrie ponctuelle par rapport à leur origine de coordonnées respective.

7. Dispositif (96) formant module de commande selon l'une des revendications 4 à 6, dans lequel le montant de la valeur de la quatrième valeur de seuil (S4) est différent du montant de la valeur de la troisième valeur de seuil (S3).

8. Dispositif (96) formant module de commande selon l'une des revendications 4 à 7, dans lequel la première fonction de convolution (513) est égale à la troisième fonction de convolution (533) et la deuxième fonction de convolution (523) est égale à la quatrième fonction de convolution (543).

9. Dispositif (96) formant module de commande selon l'une des revendications 4 à 8, dans lequel la valeur de premier seuil (S1) est égale à la valeur de troisième seuil (S3) et la valeur de deuxième seuil (52) est égal à la valeur de quatrième seuil (S4).

10. Système de chirurgie ophtalmique (1) comprenant un dispositif (96) formant module de commande de chirurgie ophtalmique selon l'une des revendications précédentes et un équipement (95) de chirurgie ophtalmique comprenant une conduite (8) de fluide d'irrigation, une première pompe à fluide (10) pour l'irrigation, une conduite (9) de fluide d'aspiration et une deuxième pompe à fluide (20) pour l'aspiration.
